# EUROPEAN PATENT SPECIFICATION

(11) **EP 3 686 286 B1**
(45) Date of publication and mention of the grant of the patent: **08.11.2023**
(21) Application number: 17925733.2
(22) Date of filing: 20.09.2017
(51) Int. Cl.: C12Q 1/68, G01N 33/574

(54) **OVARIAN CANCER BIOMARKER AND USE THEREOF**
OVARIALKARZINOM-BIOMARKER UND SEINE VERWENDUNG
BIOMARQUEUR DU CANCER DE L'OVAIRE ET SON UTILISATION

(43) Date of publication of application: 29.07.2020
(73) Proprietor: Oncolock Co., Ltd., Taipei City 11266 (TW)
(72) Inventor: TORNG, Pao-Ling, Taipei City 10487 (TW); TSAY, Yeou-Guang, Taipei City 11477 (TW); SHEN, Jenta, Tainan City 73043 (TW); CHOW, Song-Nan, Taipei City 10679 (TW); CHIEN, Chin-Hsiang, Taipei City 11156 (TW)
(74) Representative: Straus, Alexander
(86) International application number: PCT/CN2017/102407
(87) International publication number: WO 2019/056199

(56) References cited:
- WO-A2-2006/034032
- CN-A- 102 654 502
- CN-A- 102 851 388
- MIN SOON CHO ET AL: "Autocrine Effects of Tumor-Derived Complement", CELL REPORTS, vol. 6, no. 6, 1 March 2014 (2014-03-01), pages 1085-1095, XP055478089, US ISSN: 2211-1247, DOI: 10.1016/j.celrep.2014.02.014
- BJØRGE L ET AL: "Ascitic complement system in ovarian cancer", BRITISH JOURNAL OF CANCER, vol. 92, no. 5, 1 March 2005 (2005-03-01), pages 895-905, XP055815297, GB ISSN: 0007-0920, DOI: 10.1038/sj.bjc.6602334 Retrieved from the Internet: URL:https://www.ncbi.nlm.nih.gov/pmc/artic les/PMC2361909/pdf/92-6602334a.pdf>
- YAO, ZHENGLIANG: "Proteomics Approach to Identify Serum Glycoproteins as Biomarkers in Human Lung Adenocarcinoma", Dissertation, 26 November 2007 (2007-11-26), pages 1-63, XP009517915,
- PALARASAH, Y. et al.: "Generation of a C3c Specific Monoclonal Antibody and Assessment of C3c as a Putative Inflammatory Marker Derived from Complement Factor C3", Journal of Immunological Methods, vol. 362, no. 1-2, 24 September 2010 (2010-09-24), pages 142-150, XP027509601, DOI: 10.1016/j.jim.2010.09.024
- KABBAGE, M. et al.: "Protein Alterations in Infiltrating Ductal Carcinomas of the Breast as Detected by Nonequilibrium pH Gradient Electrophoresis and Mass Spectrometry", Journal of Biomedicine and Biotechnology, vol. 2008, 31 December 2008 (2008-12-31), pages 1-10, XP055317383,
- WEN, C.L. et al.: "Development of an AlphaLISA Assay to Quantify Serum Core-fucosylated E-cadherin as a Metastatic Lung Adenocarcinoma Biomarker", Journal of Proteomics, vol. 75, no. 13, 23 May 2012 (2012-05-23), pages 3963-3976, XP028497081, DOI: 10.1016/j.jprot.2012.05.015
- GAN, HUI et al.: "Advance in Researches on the Third Component of Complement(C3)", Foreign Medical Sciences (section of Clinical Biochemistry and Laboratory Medicine), vol. 25, no. 6, 30 November 2004 (2004-11-30), pages 519-521, XP009517892, ISSN: 1673-4130
- JANSSEN BERT J. C. ET AL: "Structures of complement component C3 provide insights into the function and evolution of immunity", NATURE, vol. 437, no. 7058, 1 September 2005 (2005-09-01), pages 505-511, XP055976143, London ISSN: 0028-0836, DOI: 10.1038/nature04005 Retrieved from the Internet: URL:http://www.nature.com/articles/nature0 4005>

## Description

### TECHNOLOGY FIELD

The present invention relates to a biomarker, method and assay kit for identifying and screening ovarian cancer and monitoring ovarian cancer progression.

### BACKGROUND OF THE INVENTION

Ovarian cancer is one of the most common cancers among women worldwide. It has a high prevalence rate in developed countries, and its incidence and mortality continue to rise. Undoubtedly, if ovarian cancer can be detected early, it can provide the best chance to cure and improve the survival rate of patients. Ultrasound can see images of suspected tumors, but it is impossible to distinguish whether it is ovarian cancer, and the sensitivity is not high. Due to the limitations of equipment, it is also difficult to promote large-scale screening. Currently reported ovarian cancer markers, including CA-125 (cancer antigen 125) and HE4 (epididymis protein 4), mainly serve as prognostic indicators, are not effective to serve for in ovarian cancer screening, especially for the detection of early ovarian cancer.

CA-125 is the most widely used tumor marker in the field of gynecology. But only 50-60% of patients with stage I ovarian cancer present with abnormal CA-125 expression. In addition, other malignant tumors, such as cervical cancer and breast cancer, or during pregnancy, endometriosis, pelvic inflammatory disease, etc., also have an increased level of CA-125. Therefore, CA-125 is not specific for ovarian cancer. Furthermore, about 20% of ovarian cancer patients showed low or even undetectable CA-125 expression (Moore RG et al., Curr Opin. Oncol. Sep. 22 (5): 492 -7. 2010).

The HE4 gene locates on chromosome 20. Its protein structure consists of four disulfide bonds and was first identified from male parasitoids (Hellström I. et al., CANCER RESEARCH 63, 3695-3700, July 1, 2003). It was also found expressed in many normal tissues. For example, HE4 was highly expressed in trachea and salivary glands. Although function of HE4 is still unclear, in recent years, HE4 was used to detect ovarian cancer. HE4 was found to have less false positive rate than CA-125 in detecting ovarian cancer, and could distinguish endometriosis from malignant ovarian tumors. High HE4 levels can be detected in some ovarian cancer patients who do not have CA-125 expression (Rosen DG et al., Gynecologic Oncology, November 2005 Volume 99, Issue 2, p255-526). However, the study pointed out that the amount of HE4 in the blood is only significantly increased in patients with obvious lesion and diagnosed with ovarian cancer after surgery. Therefore, HE4 is mainly used as a prognostic indicator of ovarian cancer, but not evident enough to be used as a marker for ovarian cancer screening and early stage diagnosis.

Ovarian cancer is a complex disease, including at least endometrioid ovarian cancer, clear cell ovarian cancer, serous ovarian cancer, mucinous ovarian cancer, and other types of ovarian cancer, such as ovarian carcinosarcoma, ovarian immature teratoma, ovarian mucinous and granulosa tumor, and metastatic ovarian cancer (Krukenberg, Ovarian Krukenberg's tumor), etc. To date, there are no valid available biomarkers for extensive detection of different types of ovarian cancer, and for the detection of early ovarian cancer. Cho et al. (Cell Rep 2014, Mar 27;6(6): 1085-1095; Epub 2014 Mar 6) describe a role for the complement system in enhancing cancer growth. Cancer cells secrete complement proteins that stimulate tumor growth upon activation. Complement promotes tumor growth via a direct autocrine effect that is partially independent of tumor-infiltrating cytotoxic T cells. Activated C5aR and C3aR signal through the PI3K/AKT pathway in cancer cells, and silencing the PI3K or AKT gene in cancer cells eliminates the progrowth effects of C5aR and C3aR stimulation. In patients with ovarian or lung cancer, higher tumoral C3 or C5aR mRNA levels were associated with decreased overall survival. Further, Bjørge et al. (Br J Cancer; 2005 Mar 14;92(5):895-905) characterized the C system in the intraperitoneal ascitic fluid (AF) from ovarian cancer patients. Most of the AF samples showed alternative and classical pathway haemolytic activity. The levels of C3 and C4 were similar to or in the lower normal range when compared to values in normal sera, respectively. However, elevated levels of C3a and soluble C5b-9 suggested C activation in vivo. Malignant cells isolated from the AF samples had surface deposits of C1q and C3 activation products, but not of C5b-9 (the membrane attack complex; MAC). Activation could have become initiated by anti-tumor cell antibodies that were detected in the AFs and/or by changes on tumor cell surfaces. The lack of MAC was probably due to the expression of C membrane regulators CD46, CD55 and CD59 on the tumor cells. Soluble forms of C1 inhibitor, CD59 and CD46, and the alternative pathway inhibitors factor Hand FHL-1 were present in the AF at concentrations higher than in serum samples.

Therefore, there is still a need to provide a biomarker for ovarian cancer that can be used for general screening, and especially for the early detection of ovarian cancer.

### SUMMARY OF THE INVENTION

The present invention unexpectedly finds that in ovarian cancer patients, the plasma protein complement component 3 (C3) is activated to produce a peptide fragment of about 40 kDa in size, which can be detected specifically in the plasma of patients with different types of ovarian cancer, especially at the early stage of ovarian cancer, but it is undetected or almost un-detectable in individuals free of ovarian cancer. In addition, an increased amount of this fragment can be measured in patients with progressed ovarian cancer. Therefore, the cleaved C3 protein can be used as a specific molecular marker for diagnosing ovarian cancer, especially for early diagnosis, and can also be used to monitor progression of ovarian cancer in patients with ovarian cancer, or to assess efficacy of the anti-ovarian cancer therapy. The scope of the invention shall be defined by the appended claims. A step of obtaining a sample is not a claimed part of the invention, but is only referred to for illustration of the claimed methods.

According to the invention a method is provided for detecting ovarian cancer, comprising:
detecting the amount of a cleaved complement component 3 (C3) polypeptide in a blood sample obtained from a subject to be tested to obtain a detected amount of the cleaved C3 polypeptide; comparing the detected amount of the cleaved C3 polypeptide with a normal amount of the cleaved C3 polypeptide from a normal subject, to obtain a comparison result; and determining the likelihood that the subject to be tested suffers from ovarian cancer based on the comparison result. If the comparison result shows the detected amount of the cleaved C3 polypeptide higher than the normal amount of the cleaved C3 polypeptide, the subject to be tested is determined to have ovarian cancer or be at risk of developing ovarian cancer. The cleaved C3 polypeptide is a human C3 alpha chain fragment 2.

According to another embodiment, the cleaved C3 polypeptide has an amino acid sequence corresponding to position 1321 to position 1663 of SEQ ID NO: 1; or has the amino acid sequence of SEQ ID NO: 2.

According to another embodiment, the detection of step (a) is carried out by mass spectrometry or immunoassay; or by immunoassay using an antibody that specifically binds to the cleaved C3 polypeptide having SEQ ID NO: 2.

According to another embodiment, the ovarian cancer is selected from the group consisting of endometrioid ovarian cancer, clear cell ovarian cancer, serous ovarian cancer, and mucinous ovarian cancer, or selected from the group consisting of ovarian carcinosarcoma, ovarian immature teratoma, ovarian mucinous and granulosa tumor, and metastatic ovarian cancer (Krukenberg, ovarian Krukenberg's tumor).

According to another embodiment, the ovarian cancer is selected from the group consisting of stage I ovarian cancer, stage II ovarian cancer, stage III ovarian cancer, and stage IV ovarian cancer.

According to another embodiment, a method is provided for monitoring progression of ovarian cancer in an ovarian cancer patient, the method comprising:
detecting the amount of a cleaved C3 polypeptide in a first blood sample obtained from a patient at a first time point to obtain a first detected amount of the cleaved C3 polypeptide, and detecting the amount of the cleaved C3 polypeptide in a second blood sample obtained from the patient at a second time point, wherein the second time point is later than the first time point, to obtain a second detected amount of the cleaved C3 polypeptide; comparing the first detected amount of the cleaved C3 polypeptide with the second detected amount of the cleaved C3 polypeptide to obtain a comparison result; and determining the ovarian cancer progression of the patient based on the comparison result, wherein if the comparison result shows the second detected amount of the cleaved C3 polypeptide higher than the first detected amount of the cleaved C3 polypeptide, it indicates progression of ovarian cancer. The cleaved C3 polypeptide is a human C3 alpha chain fragment 2.

According to another embodiment, the patient is subjected to an anti-ovarian cancer treatment, and the first blood sample and the second blood sample are obtained before or after the treatment or during the course of the treatment.

According to another embodiment, the method further comprises assessing efficacy of the anti-ovarian cancer treatment on the patient wherein a decrease of the amount of the cleaved C3 polypeptide after the treatment or over the course of the treatment indicates that the treatment is effective on the patient.

According to another embodiment, the cleaved C3 polypeptide has an amino acid sequence corresponding to position 1321 to position 1663 of SEQ ID NO: 1; or has the amino acid sequence of SEQ ID NO: 2.

According to another embodiment, the detection of step is carried out by mass spectrometry or immunoassay; or by immunoassay using an antibody that specifically binds to the cleaved C3 polypeptide having SEQ ID NO: 2.

According to another embodiment, the ovarian cancer is selected from the group consisting of endometrioid ovarian cancer, clear cell ovarian cancer, serous ovarian cancer, and mucinous ovarian cancer, or selected from the group consisting of ovarian carcinosarcoma, ovarian immature teratoma, ovarian mucinous and granulosa tumor, and metastatic ovarian cancer (Krukenberg, ovarian Krukenberg's tumor); or the ovarian cancer is selected from the group consisting of stage I ovarian cancer, stage II ovarian cancer, stage III ovarian cancer, and stage IV ovarian cancer.

According to another embodiment, the use of a reagent is provided that recognizes a cleaved C3 polypeptide for diagnosing ovarian cancer or monitoring progression of ovarian cancer, or for manufacturing an agent or kit for diagnosing ovarian cancer or monitoring progression of ovarian cancer. The cleaved C3 polypeptide is a human C3 alpha chain fragment 2.
According to another embodiment, the cleaved C3 polypeptide has an amino acid sequence corresponding to position 1321 to position 1663 of SEQ ID NO: 1; or the cleaved C3 polypeptide has the amino acid sequence of SEQ ID NO: 2; or the reagent is an antibody.

The details of one or more embodiments of the invention are set forth in the description below. Other features or advantages of the present invention will be apparent from the following detailed description of several embodiments, and also from the appended claims.

### BRIEF DESCRIPTION OF THE DRAWINGS

To illustrate the invention, the embodiments are illustrated in the following. However, it should be understood that the invention is not limited to the preferred embodiments shown.

In the drawings:

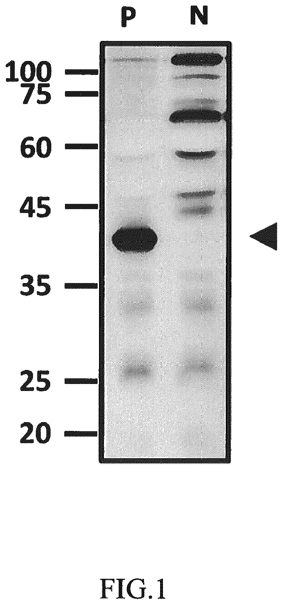

Fig. 1 shows Western blot analysis of plasma samples obtained from an ovarian cancer patient (P, patient) and a normal individual (N, normal) using antibodies against a cleaved complement component 3 (C3) protein. Proteins in plasma samples derived from the tested subjects were subjected to electrophoresis in reduced states. The number on the left, representing the molecular weight (kDa), is the position of the molecular weight marker. The arrow refers to a peptide fragment of about 40 kDa of the complement component 3 protein.

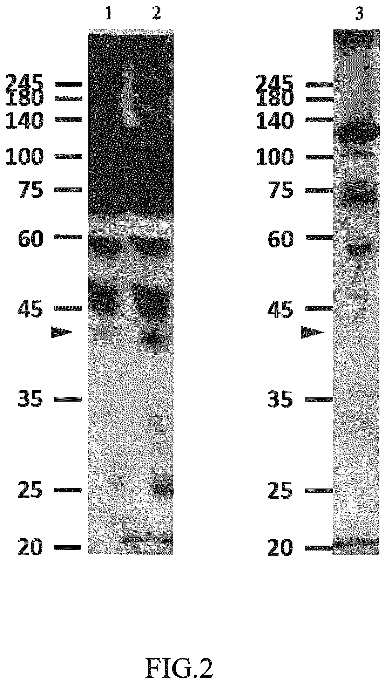

Fig. 2 shows Western blot analysis of plasma samples from an ovarian cancer patient at different time points. Lane 1 presents the analysis result of the plasma sample obtained from the ovarian cancer patient at a first time point. Lane 2 presents the analysis result of the plasma sample obtained from the same patient at one-year follow up after chemotherapy, showing an increase in the amount of the cleaved C3 protein; the patient was confirmed to suffer from ovarian cancer progression with cancer metastasis. Lane 3 presents the analysis result of the plasma sample from a normal individual, indicating that the cleaved C3 protein was not detected.

### DETAILED DESCRIPTION OF THE EMBODIMENTS

In order to provide a clear and ready understanding of the present invention, certain terms are first defined. Additional definitions are set forth throughout the detailed description. Unless defined otherwise, all technical and scientific terms used herein have the same meanings as is commonly understood by one of skill in the art to which this invention belongs.

As used herein, the articles "a" and "an" refer to one or more than one (*i.e.,* at least one) of the grammatical object of the article. By way of example, "an element" means one element or more than one element.

As used herein, the term "polypeptide" or "protein" refers to a polymer composed of amino acid residues linked via peptide bonds. The term "protein" generally refers to a polymer having a relatively large molecular weight (more amino acid residues) (e.g., containing more than 500, 600, 700, 800, 900, 1000, 1100, 1200, 1300, 1400, or 1500 amino acid residues). The term "polypeptide" or "peptide" generally refers to a polymer having a relatively small molecular weight (less amino acid residues) (e.g., containing up to 500, 400, 300, or less amino acid residues). An amino acid can be represented by three letters or one letter, as known in the art.

As used herein, the term "about" or "approximately" refers to a degree of acceptable deviation that will be understood by persons of ordinary skill in the art, which may vary to some extent depending on the context in which it is used. In general, "about" or "approximately" may mean a numeric value having a range of ± 10% around the cited value.

As used herein, the terms "subject," "individual" and "patient," used interchangeably herein, refer to any mammalian subject for whom diagnosis, prognosis, treatment, or therapy is desired, particularly humans. Other subjects may include cattle, dogs, cats, guinea pigs, rabbits, rats, mice, horses, and so on.

As used herein, the term "diagnosis" as used herein generally includes determination as to whether a subject is likely affected by a given disease, disorder or dysfunction. The skilled artisan often makes a diagnosis on the basis of one or more diagnostic indicators, i.e., a marker, the presence, absence, or amount of which is indicative of the presence or absence of the disease, disorder or dysfunction. It will be understood in the art that diagnosis does not mean determining the presence or absence of a particular disease with 100% accuracy, but rather an increased likelihood of the presence of certain disease in a subject.

As used herein, the term "treatment" refers to the application or administration of one or more active agents to a subject afflicted with a disorder, a symptom or condition of the disorder, or a progression of the disorder, with the purpose to cure, heal, alleviate, relieve, alter, remedy, ameliorate, improve, or affect the disorder, the symptom or condition of the disorder, the disabilities induced by the disorder, or the progression or predisposition of the disorder.

As used herein, "normal individual" or "control individual" may be used interchangeably to refer to an individual who is healthy and does not suffer from the disease (e.g., ovarian cancer), and may refer to a single normal individual or a group of normal individuals.

As used herein, an "aberrant amount" means an amount of an indicator that is increased compared to the amount in a subject free from a target disease (e.g., ovarian cancer) or a reference amount or a control amount. Specifically, for example, an aberrant amount can be higher than a reference or control amount by more than 10%, 20%, 30%, 40%, 50%, 60%, 70%, 80%, 90% or 100%. A reference or control amount can refer to the amount measured in normal individuals or control samples (e.g. tissues or cells free from the target disease. In this art, a range of values of normal amounts can be obtained by analyzing detected amounts of a marker in samples from a population of normal individuals using conventional detection and statistic methods.

As used herein, a biological marker is a characteristic (e.g. a protein, gene or genetic expression) that is objectively measured and evaluated as an indicator of normal or abnormal biologic processes, diseases, pathogenic processes, or responses to treatment or therapeutic interventions. Markers can include presence or absence of characteristics or patterns or collections of the characteristics which are indicative of particular biological processes. The biomarker measurement can increase or decrease to indicate a certain biological event or process. A marker is primarily used for diagnostic and prognostic purposes. However, it may be used for therapeutic, monitoring, drug screening and other purposes described herein, including evaluation the effectiveness of a cancer therapeutic.

As used herein, a biological sample to be analyzed by any of the methods described herein is a blood sample. Typically, a blood sample can be whole blood or a faction thereof e.g. serum or plasma, heparinized or EDTA treated to avoid blood clotting. Alternatively, the biological sample can be a tissue sample or a biopsy sample.

The present disclosure is based (at least in part) on the identification of a novel reliable ovarian cancer biomarker, *i.e.,* a cleaved C3 polypeptide, which is a human C3 alpha chain fragment 2. As demonstrated in the examples below, the biomarker is specifically and significantly present in blood samples of ovarian cancer patients at various stages and having various cell types, but was not detected in the blood samples of individuals suffering from other types of cancer or other diseases. Thus, the ovarian cancer detection method described herein can identify whether an individual has, is suspected of having, or is at the risk of developing ovarian cancer. The detection methods described herein can be applied to any subject (*e.g*., human female subjects), particularly as an initial, routine screening method to identify those with ovarian cancer or at the risk for progressing ovarian cancer.

### I. A cleaved C3 polypeptides can be used as a novel ovarian cancer biomarker

Complement component 3 (C3), a plasma glycoprotein, is a member of the immune system, having the molecular weight of approximately 187 kDa, and is the most abundant component in the complement system. Upon activation of the complement system, C3 can be cleaved by convertase into three fragments, including a larger C3 beta (β) fragment, and two smaller C3 alpha (α) fragments, including a C3α chain fragment 1 and a C3α chain fragment 2. Specifically, a human C3 protein comprises a full length amino acid sequence as shown in SEQ ID NO: 1 (1663 amino acids); after the cleavage, a C3β fragment is a polypeptide fragment having the amino acid sequence from position 23 to position 667, a C3α chain fragment 1 is a polypeptide fragment having the amino acid sequence from position 749 to position 954, and a C3α chain fragment 2 is a polypeptide fragment having the amino acid sequence from position 1321 to position 1663 (approximately a peptide fragment having about 342 amino acids at the C-terminus of C3 full length).

As noted herein, the present disclosure is based on the discovery that the presence and amount of a cleaved C3 polypeptide correlates with ovarian cancer occurrence and development. As used herein, the term "a cleaved C3 polypeptide" or the like (e.g. a cleaved form of C3) refers to a C3α chain fragment 2 as a result of cleavage of a full-length C3 polypeptide. Specifically, a cleaved C3 polypeptide as used herein refers to a human C3α chain fragment 2, which is a peptide fragment of about 342 amino acids at the C-terminus of a human C3 polypeptide, having a molecular weight of about 40 kDa, and more specifically, a polypeptide peptide fragment having the amino acid sequence corresponding to position 1321 to position 1663 of SEQ ID NO: 1 of a C3 protein, which is produced by hydrolysis cleavage between Arg (R) at position 1320 and Ser (S) at position 1321 of SEQ ID NO: 1 of a C3 protein. Further more specifically, a cleaved C3 polypeptide as used herein refers to a polypeptide having the amino acid sequence of SEQ ID NO: 2. It is understandable that a polypeptide may have a limited number of changes or modifications that may be made within a certain portion of the polypeptide irrelevant to its activity or function and still result in a variant with an acceptable level of equivalent or similar biological activity or function.

Such a cleaved C3 polypeptide may be prepared by a conventional method, *e.g.,* recombinant technology, using a suitable host cell (*e.g.,* E. coli, yeast, mammalian, insect, or cell-free host system) or synthesis methods. In some examples, the cleaved C3 polypeptide differs from the naturally occurring counterpart in at least one post-translational modification such as glycosylation. The cleaved form of C3 can be mixed with a pharmaceutically acceptable carrier (*e.g.,* a carrier that does not co-exist with the cleaved C3 in nature or a non-naturally occurring carrier e.g. an immunological adjuvant) to form a composition for, *e.g.*, pharmaceutical uses or inducing production of antibodies.

### II. Assays for detecting and measuring the amount of a cleaved C3 polypeptide

The presence and amount of a cleaved form of C3 polypeptide in a biological sample can be determined by any routine technology. In some embodiments, the presence and/or amount of a cleaved form of C3 polypeptide can be determined by mass spectrometry, which allows direct measurements of the analytes with high sensitivity and reproducibility. A number of mass spectrometric methods are available. Examples of mass spectrometry include, but are not limited to, matrix-assisted laser desorption ionization/time of flight (MALDI-TOF), surface-enhanced laser desorption ionisation/time of flight (SELDI-TOF), liquid chromatography-mass spectrometry (LC-MS), liquid chromatography tandem mass spectrometry (LC-MS-MS), and electrospray ionization mass spectrometry (ESI-MS). One certain example of this approach is tandem mass spectrometry (MS/MS), which involves multiple steps of mass selection or analysis, usually separated by some form of fragmentation.

In other embodiments, the presence and/or amount of a cleaved form of C3 polypeptide can be determined by an immunoassay. Examples of the immunoassays include, but are not limited to, Western blot, enzyme-linked immunosorbent assay (ELISA), radioimmunoassay (RIA), radioimmunoprecipitation assay (RIPA), immunofluorescence assay (IFA) and electrochemiluminescence (ECL).

In some examples, the presence and/or level of a cleaved form of C3 polypeptide can be determined using an agent specifically recognizes the cleaved C3, such as an antibody that specifically binds to the cleaved C3.

### (i) Antibodies that specifically bind to a cleaved C3 polypeptide

As used herein, the term "antibody" refers to an intact immunoglobulin or fragment thereof, and encompasses any polypeptide comprising an antigen-binding domain or an antigen-binding fragment that specifically binds to a particular antigen. The term includes but is not limited to monoclonal, monospecific, polyclonal, polyspecific, humanized, human, single-chain, chimeric, synthetic, recombinant, mutated, and hybrid antibodies. A suitable antibody can be prepared according to a method as known in the art. Such antibodies may not be naturally occurring (*e.g*., would not be produced in nature without human act).

An intact or complete antibody comprises two heavy chains and two light chains. Each heavy chain consists of a variable region (V_{H}) and a first, second and third constant regions (C_{H}1, C_{H}2 and C_{H}3), while each light chain consists of a variable region (V_{L}) and a constant region (C_{L}).

The term "antigen-binding domain" or "antigen-binding fragment" refers to a portion or region of an entire antibody molecule that is responsible for antigen binding. The portion of the antigen that is specifically bound or recognized by the antibody is called the "epitope." An antigen-binding domain may comprise the heavy chain variable region (V_{H}) and the light chain variable region (V_{L}); however, it does not have to comprise both. The variable region in both chains typically includes three hypervariable regions called the complementarity determining regions (CDRs). The three CDRs are interrupted by framework regions (FRs), which are more highly conserved than the CDRs. The constant regions of the heavy and light chains are not responsible for antigen binding, but exhibit various effector functions. Antibodies are classified based on the amino acid sequence of the constant region of their heavy chain. The five major classes or isotypes of antibodies are IgG, IgM, IgA, IgD and IgE, which are characterized by the presence of the constant regions of the heavy chains, gamma (γ), mu (µ), alpha (α), delta (δ) and epison (ε), respectively. Examples of antigen-binding fragments of an antibody include: (1) a Fab fragment, a monovalent fragment having the V_{L}, V_{H}, C_{L} and C_{H}1 domains; (2) a F(ab')₂ fragment, a bivalent fragment having two Fab fragments linked by a disulfide bridge at the hinge region, *i.e.* a dimer of Fab; (3) a Fv fragment having the V_{L} and V_{H} domains of a single arm of an antibody; (4) an isolated complementarity determining region (CDR); (5) a single chain Fv (scFv), a single polypeptide chain composed of a V_{H} domain and a V_{L} domain through a peptide linker; and (6) a (scFv)₂, comprising three peptide chains: two V_{H} domains linked by a peptide linker and bound by disulfide bridges to two V_{L} domains.

The term "human antibody" includes antibodies having variable and constant regions corresponding substantially to, or derived from human germline immunoglobulin sequences. The human antibodies of the invention, however, may include amino acid residues not encoded by human germline immunoglobulin sequences (*e.g*., mutations introduced by random or site-specific mutagenesis *in vitro* or by somatic mutation *in vivo*)*,* for example in the CDRs. Particularly, the human antibody may have at least one, two, three, four, five, or more positions replaced with an amino acid residue that is not encoded by the human germline immunoglobulin sequence.

In some embodiments, the antibody to be used in any of the methods described herein is an antibody that specifically binds to a cleaved C3 alpha chain fragment 2, *e.g.,* a cleaved C3 polypeptide having SEQ ID NO:2.

An antibody that "specifically binds to a target (*e.g*., a cleaved C3 polypeptide) or an epitope is a term well understood in the art, and methods to determine such specific binding are also well known in the art. A molecule is said to exhibit "specific binding" if it reacts or associates more frequently, more rapidly, with greater duration and/or with greater affinity with a particular target antigen than it does with alternative targets. Particularly, an antibody "specifically binds" to a target antigen if it binds with greater affinity, avidity, more readily, and/or with greater duration than it binds to other substances. For example, an antibody that specifically (or preferentially) binds to a cleaved C3 alpha chain fragment 2or an epitope therein is an antibody that binds this target antigen with greater affinity, avidity, more readily, and/or with greater duration than it binds to other antigens or other epitopes in the same antigen, *e.g*., the full-length C3. It is understood that "specific binding" or "preferential binding" does not necessarily require (although it can include) exclusive binding; generally, but not necessarily, reference to binding means preferential binding.

The antibody for use in the methods described herein specifically binds to a cleaved C3 alpha chain fragment 2e.g. an antibody specifically binding to a polypeptide having SEQ ID NO: 2. More specifically, the antibody that specifically recognizes a cleaved C3 alpha chain fragment 2described herein means that the antibody specifically binds to the epitope in the cleaved C3 alpha chain fragment 2, such that in the biological sample to be tested, the antibody can identify whether or not the cleaved C3 alpha chain fragment 2is present or measure the amount of the cleaved C3 alpha chain fragment 2by a conventional immunoassay method (for example, Western blotting method, e.g., through the determination of the molecular weight). In still other embodiments, the antibody that specifically recognizes a cleaved C3 alpha chain fragment 2of the present invention can specifically recognize the single amino acid residue at the N-terminus of the cleaved C3 polypeptide, for example, serine (S) at N-terminus of SEQ ID NO: 2. The antibody does not bind to the full-length C3 protein, and can be used to identify the presence or absence of the cleaved C3 polypeptide, or to further detect the content of the cleaved C3 alpha chain fragment 2.

Any of the antibodies described herein is also within the scope of the present disclosure.

### (ii) Antibody Preparation

Antibodies capable of binding a cleaved C3 as described herein can be prepared by any method known in the art. See, for example, Harlow and Lane, (1988) Antibodies: A Laboratory Manual, Cold Spring Harbor Laboratory, New York.

The antibodies described herein may be polyclonal antibodies, which can be obtained by the conventional polyclonal antibody preparation methods. Specifically, a cleaved C3 purified from human serum or the recombinant peptide prepared by genetical engineering can be used as an antigen, which is then mixed with a carrier protein, such as the bovine serum albumin (BSA) or keyhole limpet hemocyanin (KLH) to increase its immunogenicity. The antigen (or mixed with the carrier protein) is injected into an animal (e.g., a rabbit) to induce an immune response in vivo, and the immunization may be additionally performed, for example, once every two weeks for about 2-5 times in total. Subsequently, the enzyme immunoassay is carried out first to ensure a serum titer of more than 1 :4000 after immunization, and then all the blood collections can be performed. After blood collections, the blood is coagulated, and then is centrifuged to obtain the supernatant, i.e. an antiserum containing the anti-cleaved C3 polyclonal antibodies. Preferably, the antiserum can be purified, for example, by affinity chromatography (protein A/G) for purification of immunoglobulin (Ig), and further precipitation with ammonium sulfate can be used to obtain the antibody having target specificity. The ion exchange column chromatography can also be used to remove impurities from the precipitate.

In some embodiments, antibodies specifically binding to a target antigen (e.g., a cleaved C3 e.g. SEQ ID NO: 2) can be prepared by conventional hybridoma techniques. A full-length target antigen or fragment thereof can be optionally bound to a carrier protein (e.g., KLH) to immunize the host animal to produce the antibody capable of binding to the antigen. The route and schedule of immunization of the host animal are generally in keeping with established and conventional techniques for antibody stimulation and production, as further described herein. General techniques for production of mouse, humanized, and human antibodies are known in the art and are described herein. It is contemplated that any mammalian subject including humans or antibody-producing cells therefrom can be manipulated to serve as the basis for production of mammalian, including human hybridoma cell lines. Typically, the host animal is inoculated intraperitoneally, intramuscularly, orally, subcutaneously, intraplantar, and/or intradermally with an amount of immunogen, including as described herein.

Using the general somatic cell hybrid technique (Kohler, B. and Milstein, C. (1975) Nature 256:495-497 or modified from Buck, D.W., et al., In Vitro, 18:377-381 (1982)), the hybridoma can be prepared from lymphocytes and immortalized myeloma cells. Available myeloma cell lines, including, but not limited to, X63-Ag8.653 and the cells derived from the Salk Institute, Cell Distribution Center, San Diego, Calif., USA, can all be used for hybridization. In general, the technique involves the use of a fusion agent, such as polyethylene glycol, to fuse myeloma cells and lymphocytes, or by electrical means, as is well known to those skilled in the art. After fusion, cells are isolated from the fusion medium and cultured in a selective growth medium, such as hypoxanthine-aminopterin-thymidine (HAT) medium to eliminate unhybridized parent cells. Any of the media described herein, with or without serum, can be used to culture hybridomas that secrete monoclonal antibodies. The hybridomas are expanded and subcultured, and if necessary, the supernatant can be assayed for anti-immunogenic activity by conventional immunological methods (*e.g.*, radioimmunoassay, enzyme immunoassay, or fluorescence immunoassay).

The hybridoma can be used as a source of antibodies, which comprises all derivatives, progeny cells of the parental hybridoma, which produce the monoclonal antibody capable of binding to a cleaved C3. Hybridoma which produces such antibodies can be grown *in vitro* or *in vivo* using known procedures. The monoclonal antibody can be isolated from the culture medium or body fluid by conventional immunoglobulin purification procedures, such as ammonium sulfate precipitation, gel electrophoresis, dialysis, chromatography, and ultrafiltration, if applicable. Undesired activity if present, can be removed, for example, by running the preparation over adsorbents made of the immunogen attached to a solid phase and eluting or releasing the desired antibodies off the immunogen. Immunization of a host animal with a target antigen or a fragment containing the target amino acid sequence conjugated to a protein that is immunogenic in the species to be immunized, *e.g.*, keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glutaraldehyde, succinic anhydride, SOCl, or R1N=C=NR, where R and R1 are different alkyl groups, can yield a population of antibodies (*e.g*., monoclonal antibodies).

If desired, antibodies (e.g., monoclonal or polyclonal) of interest (e.g., produced from hybridoma) can be sequenced and the polynucleotide sequence can be cloned into a vector for expression or proliferation. The sequence encoding the antibody of interest can be maintained in a vector of the host cell, and the host cell can be propagated and frozen for future use. Alternatively, the polynucleotide sequence can be used in genetically engineering to form a "humanized" antibody or to improve its affinity (affinity maturity) or other characteristics of antibodies. For example, the constant region may be engineered to more resemble human constant regions to avoid immune response if the antibody is used in clinical trials and treatments in humans. It is additionally possible to genetically engineer the antibody sequence to obtain a higher affinity for the target antigen and a higher efficiency for the detection of cleaved C3. It will be appreciated by those skilled in the art that one or more polynucleotide modifications can be made to the antibody while still maintaining its binding specificity to the target antigen.

In other embodiments, intact human antibodies can be prepared using commercially available mice that are engineered to express specific human immunoglobulin. The transgenic animal is designed to produce a more desirable (e.g., intact human antibodies) or stronger immune response, and can also be used to generate humanized or human antibodies. Examples of such techniques are Xenomouse^{RTM} from Amgen, Inc. (Fremont, Calif.), and HuMAb-MouseRTM and TC Mouse^{™} from Medarex, Inc. (Princeton, N.J.). Alternatively, such antibodies can be prepared by phage expression or yeast techniques. See, e.g., U.S. Patent Nos. 5,565,332; 5,580,717; 5,733,743 and 6,265,150; and Winter et al., (1994) Annu. Rev. Immunol. 12:433-455. In addition, phage expression technique (McCafferty et al., (1990) Nature 348:552-553), derived from the immunoglobulin variable region (V) gene group of non-immunized contributors, is used to produce human antibodies and in vitro antibody fragments.

Antigen-binding fragments of an intact antibody (full-length antibody) can be prepared via routine methods. For example, F(ab')₂ fragments can be produced by pepsin digestion of an antibody molecule, and Fab fragments that can be generated by reducing the disulfide bridges of F(ab')₂ fragments.

Genetically engineered antibodies, such as humanized antibodies, chimeric antibodies, single-chain antibodies, and bi-specific antibodies, can be produced via, *e.g.,* conventional recombinant technology. In one example, DNA encoding a monoclonal antibody specific to a target antigen can be readily isolated and sequenced using conventional procedures (*e.g*., by using oligonucleotide probes that are capable of binding specifically to genes encoding the heavy and light chains of the monoclonal antibodies). The hybridoma cells serve as a preferred source of such DNA. Once isolated, the DNA may be placed into one or more expression vectors, which are then transfected into host cells such as *E. coli* cells, simian COS cells, Chinese hamster ovary (CHO) cells, or myeloma cells that do not otherwise produce immunoglobulin protein, to obtain the synthesis of monoclonal antibodies in the recombinant host cells. See, *e.g*., PCT Publication No. WO 87/04462. The DNA can then be modified, such as, by substituting the coding sequence for human heavy and light chain constant domains in place of the homologous murine sequences, Morrison et al., (1984) Proc. Nat. Acad. Sci. 81:6851, or by covalently joining to the immunoglobulin coding sequence all or part of the coding sequence for a non-immunoglobulin polypeptide. In such manner, genetically engineered antibodies, such as "chimeric" or "hybrid" antibodies, can be prepared with the binding specificity of a target antigen.

Techniques related to the preparation of "chimeric antibodies" are well known in the art. See, e.g., Morrison et al. (1984) Proc. Natl. Acad. Sci. USA 81, 6851; Neuberger et al. (1984) Nature 312, 604; and Takeda et al. (1984) Nature 314:452.

Methods for constructing humanized antibodies are also well known in the art. See, *e.g*., Queen et al., Proc. Natl. Acad. Sci. USA, 86: 10029-10033 (1989). In one example, variable regions of V_{H} and V_{L} of a parent non-human antibody are subjected to three-dimensional molecular modeling analysis following methods known in the art. Then, framework amino acid residues predicted to be important for the formation of the correct CDR structures are identified using the same molecular modeling analysis. In parallel, human V_{H} and V_{L} chains having amino acid sequences that are homologous to those of the parent non-human antibody are identified from any antibody gene database using the parent V_{H} and V_{L} sequences as search queries. Human V_{H} and V_{L} acceptor genes are then selected.

The CDR regions within the selected human acceptor genes can be replaced with the CDR regions from the parent non-human antibody or functional variants thereof. When necessary, residues within the framework regions of the parent chain that are predicted to be important in interacting with the CDR regions (see above description) can be used to substitute for the corresponding residues in the human acceptor genes.

A single-chain antibody can be prepared via recombinant technology by linking a nucleotide sequence coding for a heavy chain variable region and a nucleotide sequence coding for a light chain variable region. Preferably, a flexible linker is incorporated between the two variable regions. Alternatively, techniques described for the production of single-chain antibodies (U.S. Patent Nos. 4,946,778 and 4,704,692) can be adapted to produce a phage or yeast scFv library and scFv clones specific to a cleaved C3 can be identified from the library following routine procedures. Positive clones can be subjected to further screening to identify those that bind to a cleaved C3.

Antibodies obtained following a method known in the art and described herein can be characterized using methods well known in the art. For example, one method is to identify the epitope to which the antigen binds, or "epitope mapping." There are many methods known in the art for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, N.Y, 1999. In an additional example, epitope mapping can be used to determine the sequence to which an antibody binds. The epitope can be a linear epitope, *i.e*., contained in a single stretch of amino acids, or a conformational epitope formed by a three-dimensional interaction of amino acids that may not necessarily be contained in a single stretch (primary structure linear sequence). Peptides of varying lengths (*e.g.,* at least 4-6 amino acids long) can be isolated or synthesized (*e.g*., recombinantly) and used for binding assays with an antibody. In another example, the epitope to which the antibody binds can be determined in a systematic screening by using overlapping peptides derived from the target antigen sequence and determining binding by the antibody. According to the gene fragment expression assays, the open reading frame encoding the target antigen is fragmented either randomly or by specific genetic constructions and the reactivity of the expressed fragments of the antigen with the antibody to be tested is determined. The gene fragments may, for example, be produced by PCR and then transcribed and translated into protein *in vitro,* in the presence of radioactive amino acids. The binding of the antibody to the radioactively labeled antigen fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody in simple binding assays. In an additional example, mutagenesis of an antigen binding domain, domain swapping experiments and alanine scanning mutagenesis can be performed to identify residues required, sufficient, and/or necessary for epitope binding. For example, domain swapping experiments can be performed using a mutant of a target antigen in which various fragments of a cleaved C3 polypeptide have been replaced (swapped) with sequences from a closely related, but antigenically distinct protein (such as another member of the neurotrophin protein family). By assessing binding of the antibody to the mutant C3, the importance of the particular antigen fragment to antibody binding can be assessed. Antibodies binding to desired epitopes can be identified via epitope mapping.

### III. Diagnosis and prognosis applications of the novel ovarian cancer marker

As described herein, a cleaved C3 polypeptide after proteolytic degradation was found to be associated with the appearance and progression of ovarian cancer. Therefore, the method for screening ovarian cancer patients, monitoring ovarian cancer progression, and evaluating the effect of ovarian cancer treatment described herein can use the cleaved C3 alpha chain fragment 2as a reliable biomarker to perform. As described above, the detection method described herein can be used as an early-stage screening method for any female individual to detect whether it is likely to have ovarian cancer (especially early ovarian cancer).

The individual analyzed by this method can be any female mammalian individual, such as a human individual. In some examples, the individual can have one or more symptoms associated with ovarian cancer. In other examples, the individual may have a risk of developing ovarian cancer. In another example, the individual is asymptomatic. In yet another example, the diagnostic method described herein can be used as a routine screening method for the detection of healthy female individuals, such as women over 20, 35, 40, 45, 50, or 55 years of age in order to monitor the risk or development of potential ovarian cancer.

For the performing of the method described herein, the detection or the measurement of the amount of a cleaved C3 alpha chain fragment 2in the blood sample taken from an individual in need thereof (e.g., a human patient who does not have any symptoms of ovarian cancer, or a human patient having, suspected of having, or at risk of having ovarian cancer) is carried out by any method known in the art, such as those described herein, e.g. mass spectrometry and immunoassays. The blood sample can a plasma or a serum sample. The cleaved C3 can be detected in a quantitative or qualitative manner. Any method effective in the art to measure the presence or absence, amount, or activity of the biomarker is encompassed by the present invention. Those of ordinary skill in the art have the ability to determine which method is best suited for measuring a particular marker.

In one embodiment, the presence or absence of a cleaved C3 polypeptide is tested in the samples obtained from individuals to be tested. If the cleaved C3 alpha chain fragment 2is measured in the sample derived from the individual to be tested, it indicates that the individual is diagnosed with or at risk of developing ovarian cancer.

In some embodiments, the amount of a cleaved C3 polypeptide in the sample derived from the candidate individual can be compared to a standard value to determine whether the candidate individual has or is at risk of having ovarian cancer. The standard value represents the amount of a cleaved C3 polypeptide in the control sample. The control sample can be taken from an individual (such as a female individual) that does not have ovarian cancer. Additionally, the control sample can be a mixture of samples taken from a plurality of such individuals. Alternatively, the control individuals are matched to the candidate individual in, for example, age, gender, and/or ethnic background. Preferably, the control sample and the biological sample of the candidate individual are samples of the same species. In some embodiments, if the cleaved C3 polypeptide is measured, or the amount of the cleaved C3 polypeptide is higher than a standard value (e.g., about 10% or more above the standard value), the candidate individual may be diagnosed as having, being suspected of having, or at risk of having ovarian cancer. In some examples, using conventional methods, such as those described herein, the amount of cleaved C3 polypeptide in the control sample is not measurable in the control sample (standard value is 0, lower than the detection limit). In this example, in the detection of the biological samples derived from an individual using the same method, having the cleaved C3 polypeptide means that the individual has, is suspected of having, or is at risk of having ovarian cancer.

The method of the present invention specifically and extensively recognizes of ovarian cancers in different pathological types, as well as ovarian cancer at different stages. This biomarker is not detected in blood samples of individuals suffering from other types of cancer or other diseases. The blood sample can be detected without using complicate instruments. The subject is highly acceptable and it is suitable for large-scale screening, for early diagnosis and for continuous tracking of the condition.

Ovarian cancer as described herein includes, but is not limited to endometrioid ovarian cancer, clear cell ovarian cancer, serous ovarian cancer, mucinous ovarian cancer, and other types of ovarian cancer, for example, ovarian carcinosarcoma, ovarian immature teratoma, ovarian mucinous and granulosa tumor, and metastatic ovarian cancer (Krukenberg, ovarian Krukenberg's tumor).

Ovarian cancer as described herein includes stage I ovarian cancer, stage II ovarian cancer, stage III ovarian cancer, and stage IV ovarian cancer. The symptoms of ovarian cancer at various stages are as described in Table 1.

**Table 1: Symptoms of ovarian cancer at various stages.**

| Ovarian cancer stage | Symptoms |
|---|---|
| Stage I ovarian cancer | The lesion is confined to the unilateral or bilateral ovaries. |
| | According to whether the ascites contains malignant cells or not and the capsules of the tumors are intact or not, it is subdivided into Stages Ia, Ib, and Ic. |
| Stage II ovarian cancer | Cancer is not confined to the ovary and has invaded other tissues of the pelvic cavity. |
| | According to whether the tumor has invaded the uterus, fallopian tubes, or other tissues of the pelvic cavity, it is subdivided into Stages IIa, IIb, and IIc. |
| Stage III ovarian cancer | Tumors are not confined to unilateral or bilateral ovaries and have invaded into the abdominal cavity, the inguinal lymph nodes, or liver surface metastases. |
| | According to whether the tumor spreads into the abdominal cavity, the size of the tumor in the abdominal cavity, and whether the tumor spreads to the pelvis and the inguinal lymph nodes, it is subdivided into Stages IIIa, IIIb, and IIIc. |
| Stage IV ovarian cancer | Tumor growth invades the ovaries and has distant metastases, for example, metastasis to the chest or liver. |

In some embodiments, the amount of a cleaved C3 polypeptide in a plurality of samples derived from the candidate individuals (e.g., the ovarian cancer patients) can be measured to determine progression of the disease. For example, at least two biological samples (such as serum samples or plasma samples) can be obtained from the candidate individual at different time points. The amount of the cleaved C3 polypeptide in at least two biological samples can be measured as described herein. If the trend of the cleaved C3 polypeptide is observed to be increasing over time (e.g., the amount of the cleaved C3 polypeptide in the later obtained sample is higher than that in the sample obtained earlier), the individual is diagnosed as having, being suspected of having, or at risk of having ovarian cancer. If the individual is an ovarian cancer patient, then the increasing trend of the amount of the cleaved C3 polypeptide means progression (deterioration) of ovarian cancer.

When an individual, such as a human patient, is diagnosed as having, suspected of having, or at risk of having ovarian cancer, the individual may undergo further testing (e.g., routine physical testing, including imaging methods, such as X-ray imaging, magnetic resonance imaging (MRI), or ultrasound or surgical biopsy) to confirm the occurrence of the disease and/or to determine the stage and type of ovarian cancer.

In some embodiments, the methods described herein can further comprise treating the ovarian cancer patient to at least relieve symptoms associated with the disease. The treatment can be any conventional anti-ovarian cancer therapy, including radiation therapy, chemotherapy, and surgery. Exemplary anti-ovarian cancer chemotherapeutic agents include, but are not limited to, cytarabine (ARA-C), bevacizumab, bleomycin, carboplatinum, cisplatin, carboplatin, cyclophosphamide, etoposide (VP-16), docetaxel, doxorubicin, gemcitabine, 5-fluorouracil (5-FU), ifosfamide, Niraparib, Olaparib, paclitaxel, Rucaparib, Tamoxifene, topotecan, vinblastine, and vincristine.

In another embodiment, the method provided herein is used to assess the efficacy of ovarian cancer treatment administered in an ovarian cancer patient. For example, a plurality of biological samples can be collected from the ovarian cancer patient undergoing ovarian cancer treatment during the treatment. If the amount of the cleaved C3 alpha chain fragment 2is decreased during the treatment, i.e. the amount of the cleaved C3 alpha chain fragment 2in the blood sample obtained later is lower as compared to that of the blood sample obtained earlier, then the treatment is effective for the ovarian cancer patient. Conversely, if the amount of the cleaved C3 alpha chain fragment 2is maintained or even increased during the treatment, it means that the treatment is ineffective for the patient or the disease progresses.

As used herein, the terms "lower", "decreased", "reduced" amount or the like mean an amount less than the amount to be compared (e.g., the detected amount of marker before treatment), of which the degree of reduction has statistical significance generally recognized by those skilled in the art. In a particular embodiment, the degree of reduction may be a reduction of 20% or more, a reduction of 30% or more, a reduction of 40% or more, a reduction of 50% or more, a reduction of 60% or more, a reduction of 70% or more, a reduction of 80% or more, or a reduction of 90% or more, as compared to the detected amount of marker prior to the treatment.

As used herein, the terms "higher," "increased," "enhanced," or the like, mean an amount greater than the amount to be compared (e.g., the detected amount of marker before treatment), of which the degree of increase has statistical significance generally recognized by those skilled in the art. In a particular embodiment, the degree of increase may be an increase of 20% or more, an increase of 30% or more, an increase of 40% or more, an increase of 50% or more, an increase of 60% or more, an increase of 70% or more, an increase of 80% or more, or an increase of 90% or more, as compared to the amount to be compared.

If an ovarian cancer therapy is considered ineffective for an ovarian cancer patient, the treatment strategy can be adjusted, such as increasing the dose, frequency of treatment, or changing the more appropriate therapy.

### IV. In vitro diagnostic kit

The invention also provides a kit for performing the method, which comprises a reagent (e.g., an antibody) as described herein that specifically recognizes a cleaved C3 alpha chain fragment 2. The kit may further comprise instructions for using the kit to detect the presence or amount of the cleaved C3 alpha chain fragment 2described herein, thereby detecting ovarian cancer, and may also be used to monitor ovarian cancer progression, or to evaluate the therapeutic effect of administration to ovarian cancer patients.

### V. Use of the antibody against a cleaved C3 alpha chain fragment 2in detecting ovarian cancer

Any of the antibodies described herein can specifically recognize a cleaved C3 alpha chain fragment 2for detecting ovarian cancer. For example, the antibody can conjugately bind to a measurable marker (such as an in *vivo* imaging agent known in the art) for diagnostic purposes. Preferably, the antibody is used for in *vitro* analysis of samples from the individual to be tested by immunoassay methods, such as Western blot analysis, enzyme-linked immunosorbent assay (ELISA), radioimmunoassay (RIA), radioimmunoprecipitation (RIPA), immunofluorescence (IFA) and electrochemical fluorescence (ECL), to determine whether the cleaved C3 alpha chain fragment 2is present in the samples or to determine the content of the cleaved C3 alpha chain fragment 2.

The individual to be tested by the diagnostic method or treatment method described herein a human female individual. A human subject in need of such treatment may be a human patient with ovarian cancer, a human patient suspected of having ovarian cancer, or a human patient at risk of having ovarian cancer. The patient can be identified by conventional medical methods or by the diagnostic methods described herein.

As used herein, "effective amount" refers to the amount of each active substance that can be administered to the individual, either alone or in combination with one or more other active substances, to confer therapeutic effect on the individual. The effective amount may vary and must be judged by those skilled in the art, depending on the specific circumstances at the time of administration, the severity of the condition, respective parameters of patients, including age, physical condition, height, gender and weight, treatment schedule, the nature of the parallel therapy (if any), the specific route of administration, and other possible factors judged by the knowledge and profession of medical personnel. Such factors are well known to those of ordinary skill in the art and can be introduced without further routine experimentation. Generally, it is preferred to use the maximum dosage of the respective components or the compositions thereof, i.e., the highest safe dosage according to sensible medical judgment. It will be appreciated by those skilled in the art, however, a patient may insist on using a lower dosage or a tolerable dosage based on medical reasons, physiological responses, or other possible reasons.

The dosage is determined based on empirical considerations that are generally considered influential in dosage, such as half-life. For example, antibodies that are compatible with the human immune system, such as humanized antibodies or fully human antibodies, can extend the half-life of the antibody and prevent the antibody from being attacked by the host immune system. The frequency of administration can be determined and adjusted according to the duration of the treatment. In general, if not necessary, the frequency of administration can be adjusted according to treatment and/or inhibition, and/or improvement of ovarian cancer. In addition, a continuous release of the anti-cleaved C3 formulation may also be suitable. Various formulations and devices for maintaining the release are well known in the art.

Depending on the route of administration, the bound antibodies can be combined with a suitable pharmaceutically acceptable carrier to form a suitable formulation. Injectable compositions may contain different carriers, such as vegetable oils, dimethylacetamide, dimethylformamide, ethyl lactate, ethyl carbonate, isopropyl myristate, ethanol, and polyols (glycerin, propylene glycol, liquid polyethylene glycol and the like). For intravenous injection, the water-soluble antibody can be administered by drip, i.e. by injecting a pharmaceutical formulation containing the antibody and a physiologically acceptable excipient. The physiologically acceptable excipient can include, for example, 5% dextrose, 0.9% physiological saline, Ringer's solution, or other suitable excipients. A formulation for intramuscular use, such as a sterilized formulation, in which the antibody is a suitable soluble salt, can be dissolved by administering a pharmaceutical excipient (such as water for injection, 0.9% physiological saline, or 5% dextrose solution).

Without further elaboration, it is believed that those skilled in the art will be able to apply the invention to its fullest extent based on the above description. The following specific examples are, therefore, intended to be illustrative, and are not intended to limit the applicable scope of the invention in any way.

### Example: Specific proteolytic fragments of complement component 3 in ovarian cancer patients

First, through the modified two-dimensional gel electrophoresis system, we found that a specific fragment of complement component 3 (C3 protein) was detected in plasma from some ovarian cancer patients. Analysis of the trypsin hydrolysate by LC-MS/MS revealed that the fragment had a molecular weight of approximately 40 kDa. These data indicate that a particular fragment of the C3 protein may be a product of the proteolytic process. Further analysis of the amino acid sequence revealed that the specific fragment of the C3 protein is human C3α chain fragment 2, which is a C-terminus fragment cut between position 1,320 and position 1,321 relative to the full length human C3 protein SEQ ID NO: 1 and having the amino acid sequence of SEQ ID NO: 2.

Next, the Western blot method was performed to further understand the characteristics of the protein fragments.

### Antibody preparation

*The* preparation *method of polyclonal antibodies of the present invention comprises the steps of using a purified human cleaved C3 polypeptide fragment or the recombinant protein as an antigen, whose purity is over 95%. To increase the immunogenicity of the antigen, the peptide could be cross-linked to a suitable carrier protein, typically bovine serum albumin (BSA) or keyhole limpet hemocyanin (KLH). The antigen was injected into a rabbit to induce an immune response, so that different epitopes could induce B cells for secreting many different immunoglobulins (antibodies). Immunization was performed once every two weeks for a total of 5 immunizations, followed by enzyme immunoassay to ensure a serum titer of more than 1:4000 after immunization, and then all blood was collected. After the collected blood was coagulated, it was centrifuged to obtain the supernatant, i.e. the antiserum. Subsequently, the required antibody molecule was purified by using affinity chromatography (protein A*/*G), first to purify the immunoglobulin (Ig) and then precipitating with ammonium sulfate to obtain an antibody having target specificity. In addition, ion exchange column chromatography could be combined to remove impurities from the precipitate.*

### Western blot analysis

The protein in the plasma samples collected from human subjects was mixed with 6X Laemmli sample buffer containing 120 mM Tris-HCl, pH 6.8, 2% SDS (w/v) and 10% sucrose (w/v). For some samples, 1% 2-mercaptoethanol was added. After electrophoretic separation, proteins were transferred to Immobilon PVDF membrane (Millipore Corp.) using standard techniques. The blotted membrane was blocked with blocking buffer (containing 1% goat serum), and allowed to act at room temperature for 1 hour, followed by the action of rabbit anti-complement component 3 antibody. After washing three times with Tris-buffered physiological saline (containing 0.1% Tween 20), the membrane was treated with peroxidase-conjugated goat anti-rabbit IgG (Sigma-Aldrich). The chemical fluorescence was detected using LAS-4000 (Fujifilm, Japan).

### Result

Western blot analysis showed that a specific fragment of complement component 3 (cleaved C3 polypeptide fragment) was detected in the samples of ovarian cancer patients, approximately with 40 kDa in size. As shown in Fig. 1, cleaved C3 polypeptide fragment was detected in sample from a patient with ovarian cancer, but was not detected in sample from a normal individual. Therefore, the cleaved C3 polypeptide fragment can be used as a diagnostic indicator for ovarian cancer (specifically in plasma samples of ovarian cancer patients, but not in plasma samples of normal individuals).

To confirm the specificity of this cleaved C3 polypeptide fragment for ovarian cancer, plasma samples from human individuals with other types of cancer or other diseases were taken for Western blot analysis. The results are shown in Table 2.

**Table 2: Result of Western blot analysis using anti-cleaved C3 antibodies on plasma samples taken from human individuals with other types of cancer or other diseases.**

| Sample | Total number | Positive number | Positive percentage |
|---|---|---|---|
| Hepatocellular carcinoma (HCC) | 6 | 0 | 0% |
| Gastric cancer | 6 | 0 | 0% |
| Oral cancer | 6 | 0 | 0% |
| Colon cancer | 8 | 0 | 0% |
| Endometrial cancer | 6 | 0 | 0% |
| Diabetics mellitus | 8 | 0 | 0% |
| Heart failure | 7 | 0 | 0% |
| Normal individual | 26 | 0 | 0% |

The results showed that the presence of the cleaved C3 polypeptide fragment was not detected in the plasma samples from human individuals of other types of cancer or other diseases (detection rate was zero).

In contrast, plasma samples from human individuals with different stages of ovarian cancer were taken for Western blot analysis. The results are shown in Table 3.

**Table 3: Result of Western blot analysis using anti-cleaved C3 antibodies on plasma samples taken from human individuals having different stages of ovarian cancer.**

| Sample | Positive number | Total number | Positive percentage |
|---|---|---|---|
| Ovarian cancer stage I | 13 | 15 | 86.7% |
| Ovarian cancer stage II | 2 | 3 | 66.7% |
| Ovarian cancer stage III | 6 | 8 | 75.0% |
| Ovarian cancer stage IV | 1 | 2 | 50.0% |
| | 22 | 28 | 78.6% |

The results showed that the cleaved C3 polypeptide fragment was detected significantly associated with ovarian cancer. Among the patients without distinguished stage of ovarian cancer, 22 of the total 28 patients were detected as having the cleaved C3 polypeptide fragment. The positive percentage is as high as 78.6%, and the detection rate is nearly 80%, so it can be used as an indicator of ovarian cancer. In particular, the positive percentage in early stage (Stage I) ovarian cancer is as high as 86.7%, and the detection rate is close to 90%. This result is highly promising compared with the current existing detection techniques and could provide great help for the screening and early detection of ovarian cancer.

We further analyzed the type of ovarian cancer patients and analyzed whether the biomarker of the cleaved C3 polypeptide fragment was widely presented in different types of ovarian cancer patients. The results are shown in Table 4.

**Table 4: Result of Western blot analysis using anti-cleaved C3 antibodies on plasma samples taken from human individuals having different types of ovarian cancer.**

| Sample | Positive number | Total number | Positive percentage |
|---|---|---|---|
| Endometrioid | 6 | 7 | 85.7% |
| Clear cell | 6 | 6 | 100% |
| Serous | 7 | 12 | 58.3% |
| Mucinous | 1 | 2 | 50.0% |
| Others | 8 | 11 | 72.7% |
| Ovarian carcinosarcoma | | | |
| Ovarian immature teratoma | | | |
| Ovarian Seromucinous and granulosa tumor | | | |
| Metastatic ovarian cancer (Krukenberg, ovarian Krukenberg's tumor) | | | |
| | 28 | 38 | 73.7% |

The results showed that the cleaved C3 polypeptide fragment was significantly associated with different types of ovarian cancer, indicating that a variety of types of ovarian cancer can be screened extensively by detecting cleaved C3 polypeptide fragments. In particular, for the clear cell ovarian cancer currently with a very low detection rate, the detection rate as high as 100.0% is of great help for early diagnosis and screening of this cell type ovarian cancer.

In addition, Fig. 2 shows Western blot analysis of plasma samples from an ovarian cancer patient at different time points. Lane 1 presents the analysis result of the plasma sample obtained from the ovarian cancer patient at a first time point. Lane 2 presents the analysis result of the plasma sample obtained from the same patient at one-year follow up after chemotherapy, showing an increase in the amount of the cleaved C3 protein; the patient was confirmed to have ovarian cancer progression with cancer metastasis. Lane 3 presents the analysis result of the plasma sample from a normal individual, indicating that the cleaved C3 protein was not detected. The results showed that the cleaved C3 polypeptide fragment can be used as an indicator to monitor the therapeutic effect of ovarian cancer, and an increase in the amount indicates that ovarian cancer may be progressed.

We have confirmed that a cleaved C3 polypeptide fragment of about 40 kDa is specifically presented in ovarian cancer patients, and can be used as a biomarker for screening ovarian cancer, and for monitoring the therapeutic effect of ovarian cancer. Therefore, the present invention firstly proposes an ovarian cancer screening technique, which can specifically detect patients suffering from ovarian cancer, suspected of having ovarian cancer, or at the risk of having ovarian cancer, and can be used as an indicators at early stage (i.e. before the occurrence of any symptoms) or as a prognostic indicator after treatment. The technique of the present invention can develop a test reagent for ovarian cancer, using a cleaved C3 protein as a biomarker for screening ovarian cancer and tracking the condition after treatment. Compared with the prior arts, the technique of the present invention greatly improves the detection rate of ovarian cancer, not only can widely recognize various types of ovarian cancer, but also can recognize ovarian cancer at various stages especially with high early detection rate (close to 90%) and low false positive rate (the presence of the cleaved C3 polypeptide fragments is not detected in the sample of patients having other types of cancer or disease). This is a major breakthrough in ovarian cancer screening. Such detection test is non-invasive and can be finished by simple protein measurement of the blood samples from the individuals. It has high patient acceptance and is suitable for the development of large-scale early screening. In addition, it can also be used as an indicator to monitor the therapeutic effect of ovarian cancer. The patient's condition can be tracked continuously, so that the treatment plan can be adjusted in time for patients with poor treatment efficacy to avoid or delay the deterioration.

### Sequence information

Complement Component 3 (Homo sapiens) - Full Length (SEQ ID NO: 1) 
 60

 120

 180

 240

 300

 360

 420

 480

 540

 600

 660

 720

 780

 840

 900

 960

 1020

 1080

 1140

 1200

 1260

 1320

 1380

 1440
 
 1500

 1560

 1620

 1663
Complement Component 3 (Homo sapiens) C3α chain fragment 2 (1321-1663 a.a.) (SEQ ID NO: 2) 
SEETKENEGF TVTAEGKGQG TLSVVTMYHA KAKDQLTCNK FDLKVTIKPA PETEKRPQDA

KNTMILEICT RYRGDQDATM SILDISMMTG FAPDTDDLKQ LANGVDRYIS KYELDKAFSD

RNTLIIYLDK VSHSEDDCLA FKVHQYFNVE LIQPGAVKVY AYYNLEESCT RFYHPEKEDG

KLNKLCRDEL CRCAEENCFI QKSDDKVTLE ERLDKACEPG VDYVYKTRLV KVQLSNDFDE

YIMAIEQTIK SGSDEVQVGQ QRTFISPIKC REALKLEEKK HYLMWGLSSD FWGEKPNLSY

IIGKDTWVEH WPEEDECQDE ENQKQCQDLG AFTESMVVFG CPN
Complement Component 3 (Homo sapiens) C3α chain fragment 1 (749-954 a.a.) (SEQ ID NO: 3) 
SNLDEDIIAE ENIVSRSEFP ESWLWNVED LKEPPKNGIS TKLMNIFLKD SITTWEILAV

SMSDKKGICV ADPFEVTVMQ DFFIDLRLP YSVVRNEQVE IRAVLYNYRQ NQELKVRVEL

LHNPAFCSLA TTKRRHQQTV TIPPKSSLS VPYVIVPLKT GLQEVEVKAA VYHHFISDGV

RKSLKVVPEG IRMNKTVAVR TLDPERLGR
Complement Component 3 (Homo sapiens) C3β fragment (23-667 a.a.) (SEQ ID NO: 4)

SPMYSIITPN ILRLESEETM VLEAHDAQGD VPVTVTVHDF PGKKLVLSSE KTVLTPATNH

MGNVTFTIPA NREFKSEKGR NKFVTVQATF GTQVVEKVVL VSLQSGYLFI QTDKTIYTPG

STVLYRIFTV NHKLLPVGRT VMVNIENPEG IPVKQDSLSS QNQLGVLPLS WDIPELVNMG

QWKIRAYYEN SPQQVFSTEF EVKEYVLPSF EVIVEPTEKF YYIYNEKGLE VTITARFLYG

KKVEGTAFVI FGIQDGEQRI SLPESLKRIP IEDGSGEVVL SRKVLLDGVQ NPRAEDLVGK

SLYVSATVIL HSGSDMVQAE RSGIPIVTSP YQIHFTKTPK YFKPGMPFDL MVFVTNPDGS

PAYRVPVAVQ GEDTVQSLTQ GDGVAKLSIN THPSQKPLSI TVRTKKQELS EAEQATRTMQ

ALPYSTVGNS NNYLHLSVLR TELRPGETLN VNFLLRMDRA HEAKIRYYTY LIMNKGRLLK

AGRQVREPGQ DLVVLPLSIT TDFIPSFRLV AYYTLIGASG QREVVADSVW VDVKDSCVGS

LVVKSGQSED RQPVPGQQMT LKIEGDHGAR VVLVAVDKGV FVLNKKNKLT QSKIWDVVEK

ADIGCTPGSG KDYAGVFSDA GLTFTSSSGQ QTAQRAELQC PQPAA

## Claims

1. A method for detecting ovarian cancer, comprising:
(a) detecting the amount of a cleaved complement component 3 (C3) polypeptide in a blood sample obtained from a subject to be tested to obtain a detected amount of the cleaved C3 polypeptide;
(b) comparing the detected amount of the cleaved C3 polypeptide with a normal amount of the cleaved C3 polypeptide from a normal subject, to obtain a comparison result; and
(c) determining the likelihood that the subject to be tested suffers from ovarian cancer based on the comparison result, wherein if the comparison result shows the detected amount of the cleaved C3 polypeptide higher than the normal amount of the cleaved C3 polypeptide, the subject to be tested is determined to have ovarian cancer or be at risk of developing ovarian cancer,
wherein the cleaved C3 polypeptide is a human C3 alpha chain fragment 2.

2. The method of claim 1, wherein the cleaved C3 polypeptide
has an amino acid sequence corresponding to position 1321 to position 1663 of SEQ ID NO: 1; or
has the amino acid sequence of SEQ ID NO: 2.

3. The method of claim 1, wherein the detection of step (a) is carried out
by mass spectrometry or immunoassay; or
by immunoassay using an antibody that specifically binds to the cleaved C3 polypeptide having SEQ ID NO: 2.

4. The method of any one of claims 1 to 3, wherein the ovarian cancer is selected from the group consisting of endometrioid ovarian cancer, clear cell ovarian cancer, serous ovarian cancer, and mucinous ovarian cancer, or selected from the group consisting of ovarian carcinosarcoma, ovarian immature teratoma, ovarian mucinous and granulosa tumor, and metastatic ovarian cancer (Krukenberg, ovarian Krukenberg's tumor).

5. The method of any one of claims 1 to 4, wherein the ovarian cancer is selected from the group consisting of stage I ovarian cancer, stage II ovarian cancer, stage III ovarian cancer, and stage IV ovarian cancer.

6. A method for monitoring progression of ovarian cancer in an ovarian cancer patient, the method comprising:
(a) detecting the amount of a cleaved C3 polypeptide in a first blood sample obtained from a patient at a first time point to obtain a first detected amount of the cleaved C3 polypeptide, and detecting the amount of the cleaved C3 polypeptide in a second blood sample obtained from the patient at a second time point, wherein the second time point is later than the first time point, to obtain a second detected amount of the cleaved C3 polypeptide;
(b) comparing the first detected amount of the cleaved C3 polypeptide with the second detected amount of the cleaved C3 polypeptide to obtain a comparison result; and
(c) determining the ovarian cancer progression of the patient based on the comparison result, wherein if the comparison result shows the second detected amount of the cleaved C3 polypeptide higher than the first detected amount of the cleaved C3 polypeptide, it indicates progression of ovarian cancer,
wherein the cleaved C3 polypeptide is a human C3 alpha chain fragment 2.

7. The method of claim 6,
wherein the patient has been subjected to an anti-ovarian cancer treatment, and the first blood sample and the second blood sample have been obtained before or after the treatment or during the course of the treatment; or
further comprising assessing efficacy of the anti-ovarian cancer treatment on the patient wherein a decrease of the amount of the cleaved C3 polypeptide after the treatment or over the course of the treatment indicates that the treatment is effective on the patient.

8. The method of claim 6, wherein the cleaved C3 polypeptide
has an amino acid sequence corresponding to position 1321 to position 1663 of SEQ ID NO: 1; or
has the amino acid sequence of SEQ ID NO: 2.

9. The method of claim 6, wherein the detection of step (a) is carried out
by mass spectrometry or immunoassay; or
by immunoassay using an antibody that specifically binds to the cleaved C3 polypeptide having SEQ ID NO: 2.

10. The method of any one of claims 6 to 11,
wherein the ovarian cancer is selected from the group consisting of endometrioid ovarian cancer, clear cell ovarian cancer, serous ovarian cancer, and mucinous ovarian cancer, or selected from the group consisting of ovarian carcinosarcoma, ovarian immature teratoma, ovarian mucinous and granulosa tumor, and metastatic ovarian cancer (Krukenberg, ovarian Krukenberg's tumor); or
wherein the ovarian cancer is selected from the group consisting of stage I ovarian cancer, stage II ovarian cancer, stage III ovarian cancer, and stage IV ovarian cancer.

11. Use of a reagent that recognizes a cleaved C3 polypeptide for diagnosing ovarian cancer or monitoring progression of ovarian cancer, or for manufacturing an agent or kit for diagnosing ovarian cancer or monitoring progression of ovarian cancer,
wherein the cleaved C3 polypeptide is a human C3 alpha chain fragment 2.

12. The use of claim 11,
wherein the cleaved C3 polypeptide has an amino acid sequence corresponding to position 1321 to position 1663 of SEQ ID NO: 1; or
wherein the cleaved C3 polypeptide has the amino acid sequence of SEQ ID NO: 2; or
wherein the reagent is an antibody.

## Patentansprüche

1. Verfahren zum Nachweis von Eierstockkrebs, welches umfasst:
(a) Nachweis der Menge eines gespaltenen Polypeptids der Komplementkomponente 3 (C3) in einer Blutprobe, die von einer zu testenden Person erhalten wurde, um eine erfasste Menge des gespaltenen C3-Polypeptids zu erhalten;
(b) Vergleichen der erfassten Menge des gespaltenen C3-Polypeptids mit einer normalen Menge des gespaltenen C3-Polypeptids von einer normalen Person, um ein Vergleichsergebnis zu erhalten; und
(c) Bestimmen der Wahrscheinlichkeit, dass die zu testende Person an Eierstockkrebs leidet, basierend auf dem Vergleichsergebnis, wobei, wenn das Vergleichsergebnis zeigt, dass die erfasste Menge des gespaltenen C3-Polypeptids höher ist als die normale Menge des gespaltenen C3-Polypeptids, die zu testende Person bestimmt wird, Eierstockkrebs zu haben oder das Risiko zu haben, Eierstockkrebs zu entwickeln,
worin das gespaltene C3-Polypeptid ein menschliches C3-Alpha-Kettenfragment 2 ist.

2. Verfahren nach Anspruch 1, worin das gespaltene C3-Polypeptid
eine Aminosäuresequenz aufweist, die der Position 1321 bis Position 1663 von SEQ ID NO: 1 entspricht; oder
die Aminosäuresequenz von SEQ ID NO: 2 aufweist.

3. Verfahren nach Anspruch 1, wobei der Nachweis von Schritt (a) durchgeführt wird durch Massenspektrometrie oder Immunoassay; oder
durch Immunoassay unter Verwendung eines Antikörpers, der spezifisch an das gespaltene C3-Polypeptid mit SEQ ID NO: 2 bindet.

4. Verfahren nach einem der Ansprüche 1 bis 3, worin der Eierstockkrebs ausgewählt ist aus der Gruppe bestehend aus endometrioidem Eierstockkrebs, klarzelligem Eierstockkrebs, serösem Eierstockkrebs und muzinösem Eierstockkrebs, oder ausgewählt ist aus der Gruppe bestehend aus einem Eierstock-Karzinom, einem unreifem Eierstock-Teratom, einem muzinösem Eierstock- und Granulosazelltumor und metastasiertem Eierstockkrebs (Krukenberg-Tumor, ovarieller Krukenberg-Tumor) besteht.

5. Verfahren nach einem der Ansprüche 1 bis 4, worin der Eierstockkrebs ausgewählt ist aus der Gruppe bestehend aus Eierstockkrebs im Stadium I, Eierstockkrebs im Stadium II, Eierstockkrebs im Stadium III und Eierstockkrebs im Stadium IV.

6. Verfahren zur Überwachung des Fortschreitens von Eierstockkrebs bei einem Eierstockkrebspatienten, wobei das Verfahren umfasst:
(a) Erfassen der Menge eines gespaltenen C3-Polypeptids in einer ersten Blutprobe, die von einem Patienten zu einem ersten Zeitpunkt erhalten wurde, um eine erste erfasste Menge des gespaltenen C3-Polypeptids zu erhalten, und Erfassen der Menge des gespaltenen C3-Polypeptids in einer zweiten Blutprobe, die von dem Patienten zu einem zweiten Zeitpunkt erhalten wurde, worin der zweite Zeitpunkt später liegt als der erste Zeitpunkt, um eine zweite erfasste Menge des gespaltenen C3-Polypeptids zu erhalten;
(b) Vergleichen der ersten erfassten Menge des gespaltenen C3-Polypeptids mit der zweiten erfassten Menge des gespaltenen C3-Polypeptids, um ein Vergleichsergebnis zu erhalten; und
(c) Bestimmen des Fortschreitens des Eierstockkrebses des Patienten auf der Grundlage des Vergleichsergebnisses, wobei, wenn das Vergleichsergebnis zeigt, dass die zweite erfasste Menge des gespaltenen C3-Polypeptids höher ist als die erste erfasste Menge des gespaltenen C3-Polypeptids, dies ein Fortschreiten des Eierstockkrebses anzeigt,
worin das gespaltene C3-Polypeptid ein menschliches C3-Alphakettenfragment 2 ist.

7. Verfahren nach Anspruch 6,
wobei der Patient einer Anti-Eierstockkrebs-Behandlung unterzogen wurde und die erste Blutprobe und die zweite Blutprobe vor oder nach der Behandlung oder während des Verlaufs der Behandlung erhalten wurden; oder
ferner umfassend das Beurteilen der Wirksamkeit der Anti-Eierstockkrebs-Behandlung bei dem Patienten, wobei eine Abnahme der Menge des gespaltenen C3-Polypeptids nach der Behandlung oder während des Verlaufs der Behandlung anzeigt, dass die Behandlung bei dem Patienten wirksam ist.

8. Verfahren nach Anspruch 6, wobei das gespaltene C3-Polypeptid
eine Aminosäuresequenz aufweist, die der Position 1321 bis Position 1663 von SEQ ID NO: 1 entspricht; oder
die Aminosäuresequenz von SEQ ID NO: 2 aufweist.

9. Verfahren nach Anspruch 6, wobei der Nachweis von Schritt (a) durchgeführt wird
durch Massenspektrometrie oder Immunoassay; oder
durch Immunoassay unter Verwendung eines Antikörpers, der spezifisch an das gespaltene C3-Polypeptid mit SEQ ID NO: 2 bindet.

10. Verfahren nach einem der Ansprüche 6 bis 11,
worin der Eierstockkrebs ausgewählt ist aus der Gruppe bestehend aus endometrioidem Eierstockkrebs, klarzelligem Eierstockkrebs, serösem Eierstockkrebs und muzinösem Eierstockkrebs besteht, oder ausgewählt ist aus der Gruppe bestehend aus einem Eierstock-Karzinom, einem unreifem Eierstock-Teratom, einem muzinösem Eierstock- und Granulosazelltumor und metastasiertem Eierstockkrebs (Krukenberg-Tumor, ovarieller Krukenberg-Tumor); oder
wobei der Eierstockkrebs ausgewählt ist aus der Gruppe bestehend aus Eierstockkrebs im Stadium I, Eierstockkrebs im Stadium II, Eierstockkrebs im Stadium III und Eierstockkrebs im Stadium IV.

11. Verwendung eines Reagens, das ein gespaltenes C3-Polypeptid erkennt, zur Diagnose von Eierstockkrebs oder zur Überwachung des Fortschreitens von Eierstockkrebs, oder zur Herstellung eines Mittels oder Kits zur Diagnose von Eierstockkrebs oder zur Überwachung des Fortschreitens von Eierstockkrebs,
worin das gespaltene C3-Polypeptid ein menschliches C3-Alpha-Kettenfragment 2 ist.

12. Verwendung nach Anspruch 11,
worin das gespaltene C3-Polypeptid eine Aminosäuresequenz aufweist, die der Position 1321 bis Position 1663 von SEQ ID NO: 1 entspricht; oder
worin das gespaltene C3-Polypeptid die Aminosäuresequenz von SEQ ID NO: 2 aufweist; oder
worin das Reagenz ein Antikörper ist.

## Revendications

1. Méthode de détection du cancer de l'ovaire, comprenant:
(a) détecter la quantité d'un polypeptide C3 (composant 3 du complément) clivé dans un échantillon de sang obtenu d'un sujet à tester pour obtenir une quantité détectée de polypeptide C3 clivé;
(b) comparer la quantité détectée de polypeptide C3 clivé à une quantité normale de polypeptide C3 clivé provenant d'un sujet normal, afin d'obtenir un résultat de comparaison; et
(c) déterminer la probabilité que le sujet à tester souffre d'un cancer de l'ovaire sur la base du résultat de la comparaison, où si le résultat de la comparaison montre que la quantité détectée du polypeptide C3 clivé est supérieure à la quantité normale du polypeptide C3 clivé, le sujet à tester est considéré comme ayant un cancer de l'ovaire ou comme étant à risque de développer un cancer de l'ovaire,
dans lequel le polypeptide C3 clivé est un fragment de chaîne alpha C3 humaine 2.

2. Méthode de la revendication 1, dans laquelle le polypeptide C3 clivé a une séquence d'acides aminés correspondant à la position 1321 à la position 1663 de SEQ ID NO: 1; ou
possède la séquence d'acides aminés de SEQ ID NO: 2.

3. Méthode de la revendication 1, dans laquelle la détection de l'étape (a) est effectuée
par spectrométrie de masse ou par test immunologique; ou
par test immunologique utilisant un anticorps qui se lie spécifiquement au polypeptide C3 clivé ayant SEQ ID NO: 2.

4. Méthode de l'une des revendications 1 à 3, dans laquelle le cancer de l'ovaire est choisi dans le groupe constitué par le cancer de l'ovaire endométrioïde, le cancer de l'ovaire à cellules claires, le cancer de l'ovaire séreux et le cancer de l'ovaire mucineux, ou choisi dans le groupe constitué par le carcinosarcome de l'ovaire, le tératome immature de l'ovaire, la tumeur mucineuse et de la granulosa de l'ovaire et le cancer métastatique de l'ovaire (Krukenberg, tumeur de Krukenberg de l'ovaire).

5. Méthode de l'une des revendications 1 à 4, dans laquelle le cancer de l'ovaire est choisi dans le groupe constitué par le cancer de l'ovaire de stade I, le cancer de l'ovaire de stade II, le cancer de l'ovaire de stade III et le cancer de l'ovaire de stade IV.

6. Méthode de surveillance de la progression du cancer de l'ovaire chez une patiente atteinte d'un cancer de l'ovaire, comprenant:
(a) détecter la quantité d'un polypeptide C3 clivé dans un premier échantillon de sang obtenu d'une patiente à un premier moment pour obtenir une première quantité détectée du polypeptide C3 clivé, et détecter la quantité du polypeptide C3 clivé dans un second échantillon de sang obtenu de la patiente à un second moment, le second moment étant postérieur au premier, pour obtenir une seconde quantité détectée du polypeptide C3 clivé;
(b) comparer la première quantité détectée du polypeptide C3 clivé à la seconde quantité détectée du polypeptide C3 clivé pour obtenir un résultat de comparaison; et
(c) déterminer la progression du cancer de l'ovaire de la patiente sur la base du résultat de la comparaison. Si le résultat de la comparaison montre que la seconde quantité détectée du polypeptide C3 clivé est supérieure à la première quantité détectée du polypeptide C3 clivé, cela indique une progression du cancer de l'ovaire,
dans lequel le polypeptide C3 clivé est un fragment 2 de la chaîne alpha C3 humaine.

7. Méthode de la revendication 6,
dans laquelle la patiente a été soumise à un traitement contre le cancer de l'ovaire, et le premier échantillon de sang et le second échantillon de sang ont été obtenus avant ou après le traitement ou au cours du traitement; ou
comprenant en outre l'évaluation de l'efficacité du traitement contre le cancer de l'ovaire sur la patiente, une diminution de la quantité de polypeptide C3 clivé après le traitement ou au cours du traitement indiquant que le traitement est efficace sur la patiente.

8. Méthode de la revendication 6, dans laquelle le polypeptide C3 clivé
possède une séquence d'acides aminés correspondant à la position 1321 à la position 1663 de SEQ ID NO: 1; ou
possède la séquence d'acides aminés de SEQ ID NO: 2.

9. Méthode de la revendication 6, dans laquelle la détection de l'étape (a) est effectuée
par spectrométrie de masse ou par test immunologique; ou
par test immunologique utilisant un anticorps qui se lie spécifiquement au polypeptide C3 clivé ayant SEQ ID NO: 2.

10. Méthode de l'une des revendications 6 à 11,
dans lequel le cancer de l'ovaire est choisi dans le groupe constitué par le cancer de l'ovaire endométrioïde, le cancer de l'ovaire à cellules claires, le cancer de l'ovaire séreux et le cancer de l'ovaire mucineux, ou choisi dans le groupe constitué par le carcinosarcome de l'ovaire, le tératome immature de l'ovaire, la tumeur mucineuse et de la granulosa de l'ovaire et le cancer de l'ovaire métastatique (tumeur de Krukenberg, tumeur ovaire de Krukenberg); ou
dans lequel le cancer de l'ovaire est choisi dans le groupe constitué par le cancer de l'ovaire de stade I, le cancer de l'ovaire de stade II, le cancer de l'ovaire de stade III et le cancer de l'ovaire de stade IV.

11. Utilisation d'un réactif qui reconnaît un polypeptide C3 clivé pour diagnostiquer le cancer de l'ovaire ou surveiller la progression du cancer de l'ovaire, ou pour fabriquer un agent ou un kit pour diagnostiquer le cancer de l'ovaire ou surveiller la progression du cancer de l'ovaire,
dans lequel le polypeptide C3 clivé est un fragment 2 de la chaîne alpha C3 humaine.

12. Utilisation de la revendication 11,
dans lequel le polypeptide C3 clivé a une séquence d'acides aminés correspondant à la position 1321 à la position 1663 de SEQ ID NO: 1; ou
dans lequel le polypeptide C3 clivé a la séquence d'acides aminés de SEQ ID NO: 2; ou
dans lequel le réactif est un anticorps.
